# FASCICULE DE BREVET EUROPEEN

(11) **EP 1 023 920 B1**
(45) Date de publication et mention de la délivrance du brevet: **29.11.2006**
(21) Numéro de dépôt: 00440022.2
(22) Date de dépôt: 26.01.2000
(51) Int. Cl.: A61N 1/39

(54) **Impulsions ou série d'impulsions de défibrillation et dispositif pour les générer**
Pulse oder Pulsreihe zur Defibrillierung und Vorrichtung zur deren Erzeugung
Defibrillation pulses or pulse serie and device generating them

(30) Priorité: 27.01.1999 FR 9900989
(43) Date de publication de la demande: 02.08.2000
(73) Titulaire: Schiller Medical, 67160 Wissembourg (FR)
(72) Inventeur: Cansell, Albert, Altenstadt 67160 Wissembourg (FR); Daskalov, Ivan, 1408 Sofia (BG)
(74) Mandataire: Müller, Christoph Emanuel

(56) Documents cités:
- EP-A- 0 515 059
- WO-A-97/38753
- WO-A-98/26841
- GB-A- 2 190 296
- US-A- 4 222 386
- US-A- 4 566 457

## Description

La présente invention concerne le domaine médical, plus particulièrement la défibrillation cardiaque, et notamment la défibrillation externe ou transthoracique et la défibrillation interne, ainsi que le défibrillateur implantable, et a pour objet de nouvelles impulsions ou séries d'impulsions de défibrillation, ainsi qu'un dispositif pour générer ou produire ces dernières.

Actuellement, la grande majorité des défibrillations sont réalisées au moyen d'une impulsion monophasique positive résultant généralement de la décharge d'un condensateur à travers le patient et, le cas échéant, une inductance.

Toutefois, de nombreuses formes d'impulsions sont possibles (voir par exemple : A. Cansell, La Revue des Samu ; 1997-5, 229 à 237).

Il a notamment été proposé récemment d'interrompre la décharge du condensateur, puis de modifier le branchement pour terminer la décharge avec une polarité inversée, de manière à fournir des impulsions sous la forme d'une onde biphasique.

Ce mode de défibrillation biphasique a été décrit dans de nombreux travaux, et notamment par exemple dans le document WO 95/05215. Le principe même d'une défibrillation biphasique (avec deux ou plusieurs phases de polarités opposées) est cependant plus ancien, puisque les premières défibrillations ont été effectuées par Prevost et Batelli au moyen d'un courant alternatif sinusoïdal de 45 Hz (CR Acad. Sci. 1899 ; 129 : 1267). De même, tous les défibrillateurs utilisés dans l'ancienne URSS utilisaient et utilisent encore une décharge de condensateur oscillante, comportant deux à trois phases alternées (Negovsky et al., Resuscitation 1980; 8 : 53-67).

Par ailleurs, il a également été proposé d'utiliser, pour exciter les cellules cardiaques, des séries d'impulsions rectangulaires de même polarité délivrées à haute fréquence et délimitées par une enveloppe rectangulaire monophasique, de manière à obtenir une onde monophasique pulsée de forme rectangulaire et hachée par un signal haute fréquence (Janice L. Jones et al. , "Cellular excitation with high-frequency chopped defibrillator waveforms", Proc. IEEE, p 17 et 18, 6/1994).

WO 97/38753, EP 0 515 059 A1 et WO 98/26841 montrent des dispositifs de génération d'impulsions biphasiques en forme de dent de scie.

GB 2 190 296 A montre un défibrillateur implantable avec une impulsion monophasique qui est hachée à une fréquence élevée.

Toutefois, ces différents types d'impulsions de défibrillation connus présentent tous, soit du fait de leur nature, soit du fait du mode de réalisation des dispositifs les générant, des limitations et des inconvénients tels que, par exemple, une énergie disponible limitée, de grandes difficultés de réalisation pratique, une efficacité limitée pour une énergie disponible donnée ou encore un risque de nocivité pour le patient.

Par ailleurs, il existe constamment le besoin, compte tenu de leur caractère critique, d'améliorer l'efficacité des actes de défibrillation, notamment pour en réduire la quantité d'énergie délivrée au patient tout en garantissant un taux de réussite supérieur à celui obtenu par les techniques actuelles.

La présente invention a notamment pour but de pallier certains des inconvénients et des limitations précitées et de répondre au besoin précité.

Or, les inventeurs ont constaté, de manière inattendue et surprenante, que des impulsions ou une série d'impulsions de défibrillation constituée(s) par une onde à au moins deux phases dont les phases successives de polarités opposées sont composées chacune par une suite d'implusions élémentaires, individuelles et séparées de manière à ce que les phases sont découpées ou hachées à une fréquence plus élevée que la fréquence desdites phases successives, permettaient de surmonter au moins certains des inconvénients et limitations précités et présentaient une efficacité de défibrillation nettement supérieure aux formes d'ondes de défibrillation existantes.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
la figure 1A est une courbe intensité / temps représentant une série d'impulsions conforme à l'invention, selon un mode de réalisation de cette dernière, comportant deux, trois ou quatre phases successives A, B, C et D ;
la figure 1B est une courbe intensité / temps représentant une série d'impulsions conforme à l'invention, selon un autre mode de réalisation de cette dernière, comportant deux phases successives A et B ;
les figures 2 et 3 sont des représentations schématiques simplifiées de deux variantes de réalisation d'un dispositif de génération d'impulsions selon un premier mode de réalisation de l'invention ;
la figure 4 est une représentation simplifiée, mais plus détaillée, du dispositif représenté sur la figure 2, illustrant notamment un mode de réalisation possible des interrupteurs mis en oeuvre ;
la figure 5 est une représentation schématique simplifiée d'un second mode de réalisation d'un dispositif de génération d'impulsions selon l'invention, et,
la figure 6 est une représentation schématique simplifiée d'une autre variante de réalisation d'un dispositif de génération d'impulsions selon l'invention.

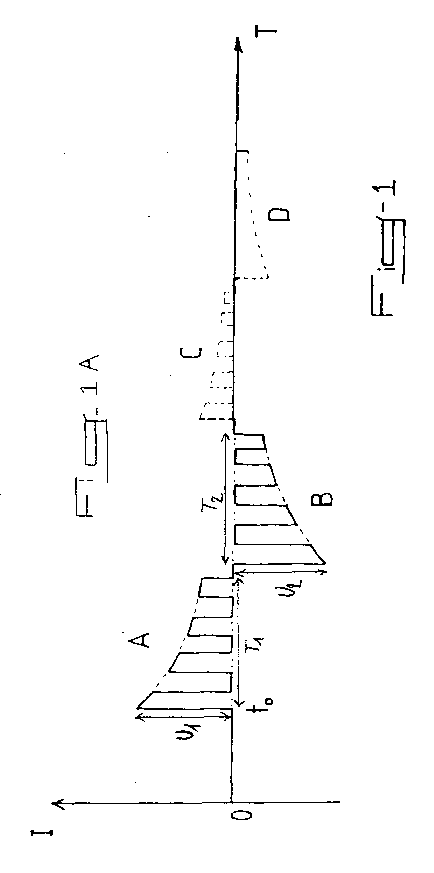

Comme le montre la figure 1 des dessins annexés, les impulsions ou la série d'impulsions de défibrillation est (sont) constituée(s) par une onde au moins biphasique dont les phases successives de polarités opposées sont découpées ou hachées à une fréquence plus élevée que la fréquence desdites phases successives.

On obtient ainsi des impulsions de défibrillation successives, de phases opposées, composées chacune par une suite d'impulsions élémentaires, individuelles et séparées, qui sont toutes délimitées par et comprises dans l'enveloppe formée par l'onde au moins biphasique. La fréquence d'occurrence et la durée des impulsions élémentaires séparées sont définies par le signal de découpage ou de hachage altérant les phases continues de ladite onde, de manière à former des phases comportant des trains d'impulsions de défibrillation successives pulsées.

Cette série d'impulsions est préférentiellement constituée par deux, (par exemple A et B), trois (par exemple A, B et C) ou éventuellement quatre (par exemple A, B, C et D) phases consécutives alternées (voir à titre d'exemples non limitatifs les séries d'impulsions représentées sur les figures 1A et 1B).

On notera qu'il est possible de faire varier aisément l'énergie de défibrillation appliquée au patient, ce quelle que soit l'énergie de l'onde multiphasique non altérée ou l'énergie emmagasinée en vue de la défibrillation, en faisant varier le facteur de forme du signal de découpage ou de hachage de ladite onde.

De manière avantageuse, la fréquence de découpage ou de hachage est au moins quatre fois supérieure à la fréquence de l'onde multiphasique.

Selon une caractéristique de l'invention, la durée de chaque phase est comprise entre 2 ms et 8 ms, préférentiellement entre 3 ms et 5 ms, la fréquence de découpage ou de hachage étant alors supérieure à 500 Hz.

Dans la pratique, la fréquence de hachage est préférentiellement supérieure à environ 1 KHz et inférieure à environ 30 KHz, préférentiellement inférieure à environ 10 KHz, les fréquences de hachage supérieures à 10 KHz, et a fortiori à 30 KHz, ne permettant plus d'obtenir pleinement les propriétés avantageuses de l'invention.

Conformément à un mode de réalisation de l'invention, correspondant en particulier à l'utilisation de décharges de condensateurs pour la génération des impulsions de défibrillation, l'enveloppe ou l'onde formant enveloppe des diverses phases se présente sous la forme d'une courbe tronquée, par exemple sous la forme d'une exponentielle tronquée (figure lA).

Selon un autre mode de réalisation de l'invention, l'enveloppe ou l'onde formant enveloppe des diverses phases successives peut avantageusement se présenter sous une forme arrondie, biphasique et asymétrique (voir à titre d'exemple la figure 1B des dessins annexés).

Dans ce second mode de réalisation de l'invention, l'onde formant enveloppe pourra être obtenue, par exemple, au moyen d'une décharge de condensateur en régime oscillant (circuit RLC), d'une décharge de condensateur à travers un filtre passe-bas (figure 1B) ou encore d'un générateur de fonction délivrant un signal de forme souhaitée.

Toutefois, au lieu de procéder au hachage de décharges ou de signaux de très haute tension, délivrés par exemple par des condensateurs, les impulsions de défibrillation conformes à l'invention peuvent également être produites aux secondaires de transformateurs amplifiant un signal haché ou pulsé au moins biphasique généré au primaire, ce qui permet de générer toute forme d'onde souhaitée.

En outre, les inventeurs ont constaté qu'il était possible d'améliorer davantage encore l'efficacité des impulsions de défibrillation conformes à l'invention en prévoyant que l'amplitude de l'onde au début de la seconde phase soit supérieure ou inférieure à l'amplitude de l'onde à la fin de la première phase, notamment dans le cas d'ondes tronquées, éventuellement en fonction de l'impédance thoracique.

Cet ajustement de l'amplitude de l'onde en début de deuxième phase peut être obtenu en utilisant par exemple deux condensateurs séparés pour générer respectivement la première et la deuxième phase.

L'expérimentation et l'évaluation plus poussées des caractéristiques globales décrites ci-dessus ont conduit à une réalisation pratique, ayant donné des résultats particulièrement intéressants. Cette réalisation particulière est décrite plus en détail, à titre d'exemple non limitatif, ci-après.

Une impulsion ou série d'impulsions de défibrillation constituée par une onde comportant deux phases consécutives alternées de polarités opposées est produite, chacune de ces phases étant obtenue au moyen de la décharge d'un condensateur, chacun de ces condensateurs (C 1 et C2) ayant une capacité de 30 µF et chacune de ces phases étant de plus découpée ou hachée à une fréquence de 5 kHz.

La première de ces phases présente à son début une tension de référence U 1 et la deuxième phase présente à son début une tension U2 dont la valeur est comprise entre environ 1/3 à 2/3 de la valeur de Ul, chacune des ces phases ayant une durée T1 et T2 d'environ 4ms.

La tension de référence U1 étant variable et déterminant l'énergie de l'impulsion, ainsi que la charge et le courant à travers le patient, dont les valeurs devront - pour que l'impulsion soit capable de défibriller - être supérieures à un seuil, appelé seuil de défibrillation.

Etant donné cependant que les patients ainsi que l'interface patient - électrodes peuvent présenter une impédance thoracique Z très fortement variable d'un cas à l'autre (entre environ 30 Ohms et 150 Ohms)-ce qui va modifier la pente des décharges exponentielles qui constituent les enveloppes des deux phases ainsi que l'énergie, le courant moyen à travers le patient, la charge électrique délivrée et en définitive l'efficacité de la défibrillation - les inventeurs ont recherché un moyen pour tenir compte de ces variations d'impédance thoracique en la mesurant au cours du choc de défibrillation et en agissant sur la durée de chacune des impulsions ou phases ou, le cas échéant, de l'une des deux seulement, pour tenter de compenser ces variations de caractéristiques et d'efficacité.

La mesure de l'impédance thoracique est réalisée en mesurant la tension aux bornes du patient au moyen de mesures successives effectuées environ toutes les 500 µs (aux instants où le courant est présent) et en calculant pour chaque paire de points successifs la résistance correspondante, connaissant la valeur du condensateur fournissant l'impulsion.

Disposant de l'impédance thoracique Z, on va agir sur la durée de chaque phase. Pour cela, si on reprend l'exemple de la réalisation préférentielle qui précède, la solution proposée par les inventeurs pour rendre les valeurs de T1 et de T2 variables et réglables en fonction de Z (impédance propre du patient + impédance de l'interface patient / électrodes du défibrillateur) consiste d'abord à associer la valeur de 4 ms utilisée plus haut pour T1 et T2, à une valeur moyenne de Z d'environ 80 Ohms.

Si Z a une valeur plus basse que 80 Ohms pour une des phases ou les deux, alors T1 et/ou T2 vont être diminuées, sans pour cela descendre en-dessous d'une valeur de 3 ms, pour ne pas descendre en-dessous de la limite inférieure imposée par la constante de temps de la cellule myocardique.

Si Z a une valeur supérieure à 80 Ohms pour une des phases ou les deux, alors T1 et/ou T2 vont être augmentées, sans toutefois dépasser une valeur de 5 ms pour ne pas aller au-delà de la limite supérieure imposée par la constante de temps de la cellule myocardique.

Il est important de noter que cette régulation de la durée de la première et/ou de la seconde phase ou impulsion en fonction de la valeur de l'impédance thoracique Z mesurée en cours de défibrillation, ne consiste pas en une simple compensation pour avoir une énergie constante, mais en une régulation des paramètres des impulsions ou phases de défibrillation pour rester dans une plage optimale de leurs caractéristiques sur le plan physiologique.

Le tableau ci-après indique des valeurs approximatives trouvées comme efficaces pour une telle régulation de T1 et T2 en fonction de Z :

| Z en Ohm | T1 ou T2 en ms |
|---|---|
| 30 | 3 |
| 40 | 3 |
| 60 | 3,5 |
| 80 | 4 |
| 100 | 4,5 |
| 120 | 5 |
| 150 | 5 |

Il convient de noter que ces valeurs ne sont données qu'à titre indicatif et non limitatif, sur la base des résultats des expériences des inventeurs effectuées jusqu'à présent.

Les dispositions de réglage et de régulation décrites ci-dessus pourront bien entendu également être appliquées dans le cadre de procédés de défibrillation à d'autres structures de dispositifs défibrillateurs, par exemple du type ne comportant qu'un condensateur au lieu des deux condensateurs C1 et C2 pour le stockage de l'énergie, ou d'ondes biphasiques non découpées.

La présente invention a également pour objet un dispositif de génération d'impulsions de défibrillation du type décrit ci-dessus, formant partie constitutive d'un défibrillateur.

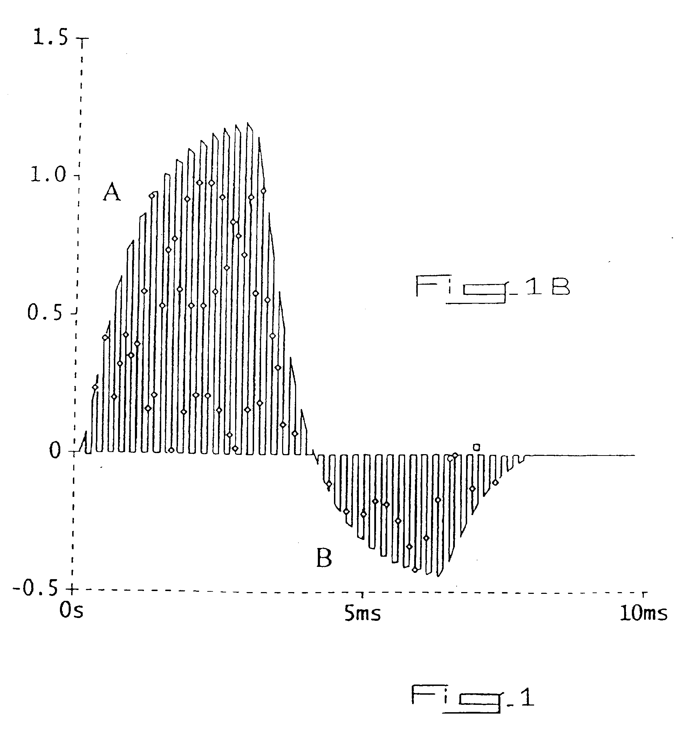

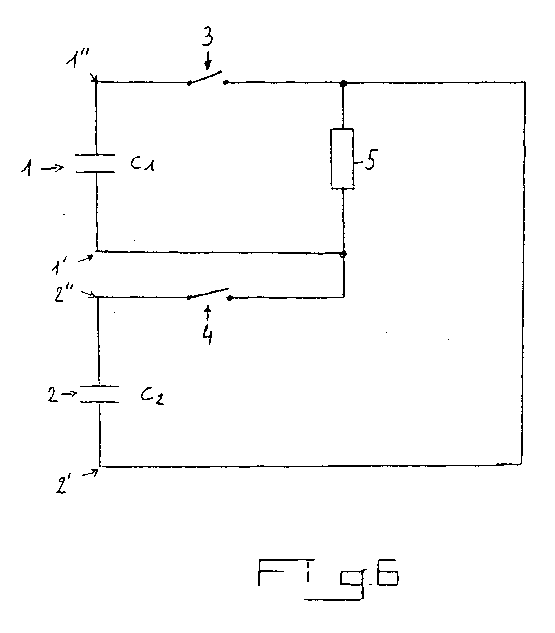

Comme le montrent les figures 2, 3 et 6 des dessins annexés, ledit dispositif peut être essentiellement constitué, d'une part, par deux condensateurs 1 et 2, présentant ou non deux bornes communes 1' et 2' ou reliées entre elles, destinés au stockage de l'énergie électrique de défibrillation, et, d'autre part, par au moins deux interrupteurs ou commutateurs 3 et 4 commandés par un circuit de commande 6, assurant chacun la connexion alternée de l'autre borne ou de l'une 1", 2" des bornes de chacun des deux condensateurs 1 et 2 avec le patient 5 et actionnés successivement de manière à produire des décharges partielles des condensateurs respectifs concernés 1 et 2 générant une série d'impulsions sous forme d'une onde au moins biphasique dont les phases successives de polarités opposées sont découpées ou hachées à une fréquence plus élevée que la fréquence desdits phases successives.

Selon une première variante de réalisation de l'invention, représentée à la figure 2 des dessins annexés, les bornes 1" et 2" des condensateurs 1 et 2 déchargés alternativement présentent des polarités opposées.

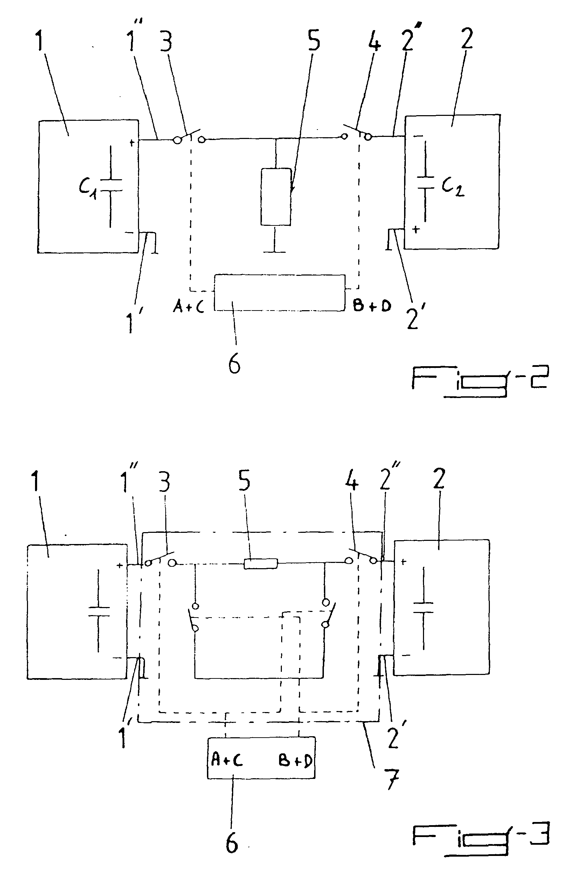

Conformément à une seconde variante de réalisation de l'invention, représentée à la figure 3 des dessins annexés, les bornes 1" et 2" des condensateurs 1 et 2 déchargés alternativement présentent des polarités identiques, les impulsions résultantes étant appliquées au patient 5 à travers un montage en pont 7 pouvant délivrer des impulsions de phases opposées.

Les condensateurs 1 et 2 pourront soit présenter un point commun, soit être complètement séparés et indépendants en fonction de l'utilisation envisagée et de la construction retenue pour l'appareil ou le dispositif défibrillateur.

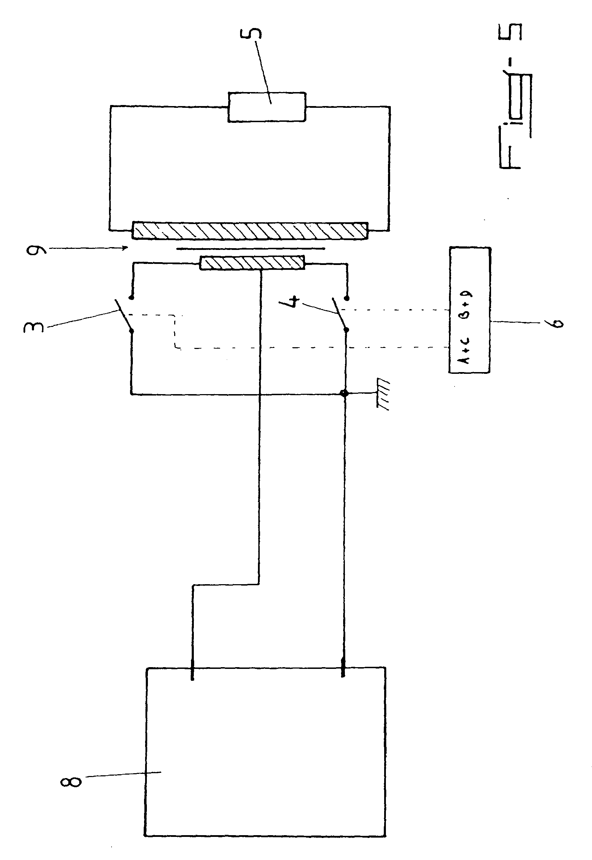

Selon un autre mode de réalisation de l'invention, représenté à la figure 5 des dessins annexés et permettant notamment la réalisation de formes d'ondes ou d'enveloppes variées, le dispositif de génération d'impulsions de défibrillation du type précité peut être principalement constitué par un générateur d'ondes à basse tension 8 relié au milieu du primaire d'un transformateur élévateur de tension 9 dont le secondaire est relié au patient 5, les deux extrémités dudit primaire pouvant être alternativement reliées à la masse par l'intermédiaire d'interrupteurs ou de commutateurs 3 et 4.

Comme le montrent les figures 2 à 5 des dessins annexés, le hachage de l'onde est réalisé par l'actionnement des interrupteurs ou commutateurs 3 et 4 au moyen d'un signal en créneaux dont la fréquence et le facteur de forme est avantageusement variable, délivré par un circuit de commande 6 intégrant un générateur de signaux correspondant, des circuits d'horloge ou analogues.

Les interrupteurs 3 et 4 assurent une fonction de hachage et d'inversion.

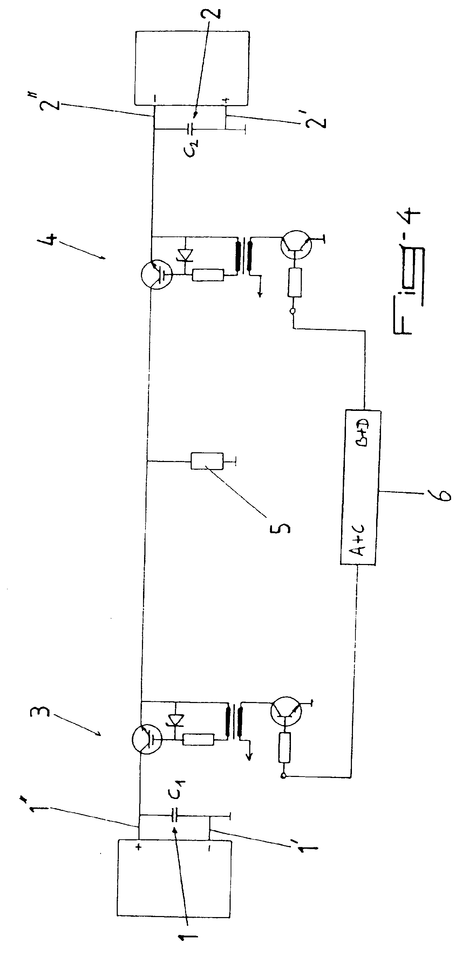

Comme le montre la figure 4 des dessins annexés, les interrupteurs 3 et 4 peuvent, par exemple, être chacun constitué par un circuit à transistor connu sous la désignation IGBT, ou par plusieurs circuits de ce type montés en série et/ou en parallèle, ce en fonction des tensions de charge des condensateurs 1 et 2 et des tensions maximales et courants des impulsions de défibrillation appliquées au patient.

Le dispositif de génération présentera également, le cas échéant, les moyens nécessaires pour réaliser la mesure d'impédance thoracique en cours de défibrillation et pour réguler en conséquence les décharges des condensateurs 1 et 2 et/ou les durées des phases successives.

Les résultats expérimentaux de défibrillations effectuées par les inventeurs sur des animaux, au moyen des différentes formes d'impulsions de défibrillation évoquées ci-dessus, sont résumés dans le tableau ci-dessous.

Pour la forme d'impulsion traditionnelle monophasique on a posé une énergie de référence de valeur 100 nécessaire et suffisante pour obtenir une défibrillation efficace. Les autres formes d'impulsion sont données en valeurs relatives, par rapport à la valeur de référence précitée, également nécessaires et suffisantes pour réaliser une défibrillation efficace.

| | |
|---|---|
| Onde monophasique | 100 |
| Onde biphasique (non découpée) | 65 |
| Onde biphasique découpée à 5 KHz conformément à la présente invention | 30 à 40 |

Il peut donc être estimé, sur la base des résultats ci-dessus, qu'un procédé de défibrillation, notamment transthoracique, appliqué à l'être humain et mettant en oeuvre des impulsions ou des séries d'impulsions selon la présente invention, par exemple délivrées par un dispositif tel que décrit précédemment, présentera, par rapport aux procédés connus, une amélioration similaire.

De même, les avantages de réduction d'énergie obtenus dans l'application particulière de la défibrillation externe, dont les résultats sont décrits ci-dessus, peuvent également être observés dans une proportion similaire dans le cas de la défibrillation interne et du défibrillateur implantable, en mettant en oeuvre les principes de la présente invention.

## Revendications

1. Impulsions ou série d'impulsions de défibrillation, qui sont constituées par une onde à au moins deux phases dont les phases successives de polarités opposées sont composées chacune par une suite d'impulsions élémentaires, individuelles et séparées de manière à ce que les phases sont découpées ou hachées à une fréquence plus élevée que la fréquence desdites phases successives.

2. Impulsions ou série d'impulsions de défibrillation selon la revendication 1, **caractérisées en ce qu'**elles sont constituées par deux phases consécutives alternées de polarités opposées.

3. Impulsions ou série d'impulsions de défibrillation selon la revendication 1, **caractérisées en ce qu'**elles sont constituées par trois phases consécutives alternées.

4. Impulsions ou série d'impulsions de défibrillation selon l'une quelconque des revendications 1 à 3, **caractérisées en ce que** la fréquence de découpage ou de hachage est au moins quatre fois supérieure à la fréquence de l'onde multiphasique.

5. Impulsions ou série d'impulsions de défibrillation selon l'une quelconque des revendications 1 à 4, **caractérisées en ce que** la durée de chaque phase est comprise entre 2 ms et 8 ms, préférentiellement entre 3 ms et 5 ms, et **en ce que** la fréquence de découpage ou de hachage est supérieure à 500 Hz.

6. Impulsions ou série d'impulsions de défibrillation selon la revendication 5, **caractérisées en ce que** la fréquence de hachage est supérieure à environ 1 KHz et inférieure à environ 30 KHz.

7. Impulsions ou série d'impulsions de défibrillation selon l'une quelconque des revendications 1 à 6, **caractérisées en ce que** l'enveloppe des diverses phases se présente sous la forme d'une courbe tronquée, par exemple sous la forme d'une exponentielle tronquée.

8. Impulsions ou série d'impulsions de défibrillation selon l'une quelconque des revendications 1 à 6, **caractérisées en ce que** l'enveloppe ou l'onde formant enveloppe des diverses phases successives se présente sous une forme arrondie, biphasique et asymétrique.

9. Impulsions ou série d'impulsions de défibrillation selon l'une quelconque des revendications 1 à 8, **caractérisées en ce que** l'amplitude de l'onde au début de la seconde phase est supérieure ou inférieure à l'amplitude de l'onde à la fin de la première phase.

10. Impulsions ou série d'impulsions de défibrillation selon l'une quelconque des revendications 1 à 9, **caractérisées en ce que** la valeur de la tension (U2) en début de seconde phase est comprise entre environ 1/3 à 2/3 de la valeur de la tension (U1) en début de première phase.

11. Impulsions ou série d'impulsions de défibrillation selon l'une quelconque des revendications 1 à 10, **caractérisées en ce que** la durée (T1, T2) de la première et/ou de la seconde phase ou impulsion est régulée ou réglée en fonction de la valeur de l'impédance thoracique (Z) mesurée en cours de défibrillation.

12. Impulsions ou série d'impulsions de défibrillation selon l'une quelconque des revendications 1 à 11, **caractérisées en ce que** le facteur de forme du signal de découpage ou de hachage de l'onde de défibrillation varie.

13. Impulsions ou série d'impulsions de défibrillation selon la revendication précédente, **caractérisées en ce que** le facteur de forme du signal de découpage ou de hachage de l'onde de défibrillation varie en fonction de l'énergie de défibrillation que l'on veut appliquer au patient.

14. Dispositif de génération d'impulsions de défibrillation permettant de générer les impulsions selon l'une quelconque des revendications 1 à 13 qui est
constitué d'une part par au moins un condensateur destiné au stockage de l'énergie électrique de défibrillation et d'autre part par au moins deux interrupteurs, ou commutateurs commandés par un circuit de commande,
ou principalement constitué par un générateur d'ondes à basse tension (8) relié au milieu du primaire d'un transformateur élévateur de tension (9) dont le secondaire adapté à être relié au patient (5), les deux extrémités dudit primaire pouvant être alternativement reliées à la masse par l'intermédiaire d'interrupteurs ou de commutateurs (3 et 4),
de manière à générer une série d'impulsions sous forme d'une onde au moins biphasique dont les phases successives de polarités opposées sont composées chacune par une suite d'implusions élémentaires, individuelles et séparées de manière à ce que les phases sont découpées ou hachées à une fréquence plus élevée que la fréquence desdites phases successives.

15. Dispositif selon la revendication précédente **caractérisé en ce qu'**il comporte deux condensateurs et **en ce que** les interrupteurs ou commutateurs commandés par le circuit de commende assurent la connexion alternée des bornes de chacun des deux condensateurs avec les électrodes appliquées sur le patient et sont actionnés successivement de manière à produire des décharges partielles des condensateurs respectifs concernés.

16. Dispositif selon la revendication 14 ou 15, **caractérisé en ce que** les bornes (1" et 2") des condensateurs (1 et 2) déchargés alternativement présentent des polarités opposées.

17. Dispositif selon la revendication 14 ou 15, **caractérisé en ce que** les bornes (1" et 2") des condensateurs (1 et 2) déchargés alternativement présentent des polarités identiques, les impulsions résultantes étant adaptées à être appliquées au patient (5) à travers un montage en pont (7) pouvant délivrer des impulsions de phases opposées.

18. Dispositif selon l'une quelconque des revendications 14 à 17 **caractérisé en ce qu'**il comporte des moyens pour faire varier le facteur de forme du signal de découpage ou de hachage de ladite onde.

19. Dispositif selon la revendication précédente **caractérisé en ce qu'**il comporte des moyens pour faire varier le facteur de forme du signal de découpage ou de hachage de ladite onde afin de faire varier l'énergie de défibrillation appliquée au patient.

20. Dispositif selon la revendication précédente **caractérisé en ce qu'**il est constitué d'une part par au moins un condensateur destiné au stockage de l'énergie électrique de défibrillation et d'autre part par au moins deux interrupteurs ou commutateurs commandés par un circuit de commande, de manière à générer une série d'impulsions sous forme d'une onde au moins biphasique dont les phases successives de polarités opposées sont découpées ou hachées à une fréquence plus élevée que la fréquence desdites phases successives selon un facteur de forme qui varie.

## Claims

1. Defibrillation pulses or pulse Defibrillation series which consist of a wave with at least two phases, of which the successive phases with opposite polarities each consist of a sequence of individual separated elementary pulses in such a manner that the phases are segmented or cut at a higher frequency than the frequency of the successive phases.

2. Defibrillation pulses or pulse series according to Claim 1, **characterized in that** they consist of two consecutive alternating phases with opposite polarities.

3. Defibrillation pulses or pulse series according to Claim 1, **characterized in that** they consist of three consecutive alternating phases.

4. Defibrillation pulses or pulse series according to any one of Claims 1 to 3, **characterized in that** the segmenting or cutting frequency is at least four times higher than the frequency of the multiphase wave.

5. Defibrillation pulses or pulse series according to any one of Claims 1 to 4, **characterized in that** the duration of each phase lies between 2 ms and 8 ms, preferably between 3 ms and 5 ms, and **in that** the segmenting or cutting frequency is higher than 500 Hz.

6. Defibrillation pulses or pulse series according to Claim 5, **characterized in that** the cutting frequency is higher than about 1 KHz and lower than about 30 KHz.

7. Defibrillation pulses or pulse series according to any one of Claims 1 to 6, **characterized in that** the envelope of the various phases is presented in the form of a truncated curve, for example in the form of a truncated exponential.

8. Defibrillation pulses or pulse series according to any one of Claims 1 to 6, **characterized in that** the envelope or the wave forming the envelope of the various successive phases is presented in a rounded, biphase and asymmetric form.

9. Defibrillation pulses or pulse series according to any one of Claims 1 to 8, **characterized in that** the amplitude of the wave at the beginning of the second phase is higher or lower than the amplitude of the wave at the end of the first phase.

10. Defibrillation pulses or pulse series according to any one of Claims 1 to 9, **characterized in that** the value of the voltage (U2) at the beginning of the second phase lies between about 1/3 to 2/3 of the value of the voltage (U1) at the beginning of the first phase.

11. Defibrillation pulses or pulse series according to any one of Claims 1 to 10, **characterized in that** the duration (T1, T2) of the first and/or the second phase or pulse is regulated or controlled as a function of the value of the thoracic impedance (Z) measured in the course of the defibrillation.

12. Defibrillation pulses or pulse series according to any one of Claims 1 to 11, **characterized in that** the form factor of the segmenting or cutting signal of the defibrillation wave varies.

13. Defibrillation pulses or defibrillation pulse series according to the preceding claim, **characterized in that** the form factor of the segmenting or cutting signal of the defibrillation wave varies as a function of the defibrillation energy which is to be applied to the patient.

14. Device for generating defibrillation pulses for generating the pulses according to any one of Claims 1 to 13, which, on the one hand, consists of at least one capacitor for storing the electrical defibrillation energy and, on the other hand, of at least two interrupters or switches controlled by a control circuit,
or mainly consists of a generator for low voltage waves (8) connected at the primary of a voltage step-up transformer (9), the secondary of which is adapted to be connected to the patient (5), wherein the two ends of the primary can be alternately linked to earth via the interrupters or the switches (3 and 4),
to generate a series of pulses in the form of an at least biphase wave, of which the successive phases with opposite polarities in each case consist of a sequence of individual separated elementary pulses in such a manner that the phases are segmented or cut at a higher frequency than the frequency of the successive phases.

15. Device according to the preceding claim, **characterized in that** it comprises two capacitors and **in that** the interrupters or switches controlled by the control circuit ensure alternating connections of the terminals of each of the two capacitors with the electrodes applied to the patient and are successively operated so as to produce partial discharges of the capacitors concerned in each case.

16. Device according to Claim 14 or 15, **characterized in that** the terminals (1* and 2*) of the capacitors (1 and 2) discharged alternately have opposite polarities.

17. Device according to Claim 14 or 15, **characterized in that** the terminals (1* and 2*) of the capacitors (1 and 2) discharged alternately have identical polarities, wherein the resultant pulses are adapted to be applied to the patient (5) via a bridge circuit (7) which can deliver pulses with opposite phases.

18. Device according to any one of Claims 14 to 17, **characterized in that** it comprises means for varying the form factor of the segmenting or cutting signal of said wave.

19. Device according to the preceding claim, **characterized in that** it comprises means for varying the form factor of the segmenting or cutting signal of the wave for varying the defibrillation energy applied to the patient.

20. Device according to the preceding claim, **characterized in that** it consists, on the one hand, of at least one capacitor for storing the electrical defibrillation energy and, on the other hand, of at least two interrupters or switches controlled by a control circuit, so as to generate a pulse series in the form of an at least biphase wave, of which the successive phases with opposite polarities are segmented or cut at a higher frequency than the frequency of the successive phases, according to a form factor which varies.

## Patentansprüche

1. Pulse oder Pulsreihe zur Defibrillierung, die aus einer Welle mit mindestens zwei Phasen bestehen, deren aufeinanderfolgende Phasen mit entgegengesetzten Polaritäten jeweils aus einer Folge von einzelnen getrennten Elementarpulsen so zusammengesetzt sind, dass die Phasen mit einer höheren Frequenz als der Frequenz der aufeinanderfolgenden Phasen aufgeteilt oder zerschnitten sind.

2. Pulse oder Pulsreihe zur Defibrillierung nach Anspruch 1, **dadurch gekennzeichnet, dass** sie aus zwei aufeinanderfolgenden abwechselnden Phasen mit entgegengesetzten Polaritäten bestehen.

3. Pulse oder Pulsreihe zur Defibrillierung nach Anspruch 1, **dadurch gekennzeichnet, dass** sie aus drei aufeinanderfolgenden abwechselnden Phasen bestehen.

4. Pulse oder Pulsreihe zur Defibrillierung nach einem beliebigen der Ansprüche 1 bis 3, **dadurch gekennzeichnet, dass** die Frequenz der Aufteilung oder der Zerschneidung mindestens viermal höher als die Frequenz der Mehrphasenwelle ist.

5. Pulse oder Pulsreihe zur Defibrillierung nach einem beliebigen der Ansprüche 1 bis 4, **dadurch gekennzeichnet, dass** die Dauer jeder Phase zwischen 2 ms und 8 ms und vorzugsweise zwischen 3 ms und 5 ms beträgt und dass die Frequenz der Aufteilung oder der Zerschneidung höher als 500 Hz ist.

6. Pulse oder Pulsreihe zur Defibrillierung nach Anspruch 5, **dadurch gekennzeichnet, dass** die Frequenz der Zerschneidung höher als etwa 1 kHz und niedriger als etwa 30 kHz ist.

7. Pulse oder Pulsreihe zur Defibrillierung nach einem beliebigen der Ansprüche 1 bis 6, **dadurch gekennzeichnet, dass** die Umhüllende der verschiedenen Phasen die Form einer abgeschnittenen Kurve beispielsweise die Form einer abgeschnittenen Exponentialkurve darstellt.

8. Pulse oder Pulsreihe zur Defibrillierung nach einem beliebigen der Ansprüche 1 bis 6, **dadurch gekennzeichnet, dass** die Umhüllende oder die Welle, die die Umhüllende der verschiedenen aufeinanderfolgenden Phasen bildet, in abgerundeter, zweiphasiger und asymmetrischer Form dargestellt ist.

9. Pulse oder Pulsreihe zur Defibrillierung nach einem beliebigen der Ansprüche 1 bis 8, **dadurch gekennzeichnet, dass** die Amplitude der Welle zu Beginn der zweiten Phase höher oder niedriger als die Amplitude der Welle am Ende der ersten Phase ist.

10. Pulse oder Pulsreihe zur Defibrillierung nach einem beliebigen der Ansprüche 1 bis 9, **dadurch gekennzeichnet, dass** der Wert der Spannung (U2) zu Beginn der zweiten Phase zwischen 1/3 bis 2/3 des Wertes der Spannung (U1) zu Beginn der ersten Phase beträgt.

11. Pulse oder Pulsreihe zur Defibrillierung nach einem beliebigen der Ansprüche 1 bis 10, **dadurch gekennzeichnet, dass** die Dauer (T1, T2) der ersten und/oder der zweiten Phase oder des zweiten Pulses in Abhängigkeit von dem Wert des im Verlauf der Defibrillierung gemessenen Brustkorbwiderstandes (Z) geregelt oder reguliert wird.

12. Pulse oder Pulsreihe zur Defibrillierung nach einem beliebigen der Ansprüche 1 bis 11, **dadurch gekennzeichnet, dass** der Formfaktor des Signals zur Aufteilung oder zur Zerschneidung der Defibrillierungswelle variiert.

13. Pulse oder Pulsreihe zur Defibrillierung nach dem vorhergehenden Anspruch, **dadurch gekennzeichnet, dass** der Formfaktor des Signals zur Aufteilung oder Zerschneidung der Defibrillierungswelle in Abhängigkeit von der Defibrillierungsenergie variiert, die an den Patienten angelegt werden soll.

14. Vorrichtung zur Erzeugung von Impulsen zur Defibrillierung zum Erzeugen der Pulse nach einem beliebigen der Ansprüche 1 bis 13, die einerseits aus mindestens einem Kondensator zum Speichern der elektrischen Defibrillierungsenergie und andererseits aus mindestens zwei Unterbrechern oder Umschaltern besteht, die von einer Steuerschaltung gesteuert werden;
oder hauptsächlich aus einem Niederspannungswellengenerator (8) besteht, der an die Primärwicklung eines Aufspannungstransformators (9) angeschlossen ist, dessen Sekundärwicklung dafür ausgebildet ist, mit dem Patienten (5) verbunden zu werden, wobei die zwei Enden der Primärwicklung abwechselnd über die Unterbrecher oder Umschalter (3 und 4) mit Erde verbunden werden können,
zum Erzeugen einer Reihe von Pulsen in der Form einer mindestens zweiphasigen Welle, deren aufeinanderfolgende Phasen mit entgegengesetzten Polaritäten jeweils so aus einer Folge von einzelnen, getrennten Elementarpulsen zusammengesetzt sind, dass die Phasen mit einer höheren Frequenz aufgeteilt oder zerschnitten werden, als der Frequenz der aufeinanderfolgenden Phasen.

15. Vorrichtung nach dem vorhergehenden Anspruch, **dadurch gekennzeichnet, dass** sie zwei Kondensatoren umfasst und dass die von der Steuerschaltung gesteuerten Unterbrecher oder Umschalter die abwechselnde Verbindung von Anschlüssen jedes der zwei Kondensatoren mit den an den Patienten angelegten Elektroden sicherstellen und nacheinander so betätigt werden, dass sie teilweise Entladungen der jeweils betroffenen Kondensatoren erzeugen.

16. Vorrichtung nach Anspruch 14 oder 15, **dadurch gekennzeichnet, dass** die Anschlüsse (1' und 2') der abwechselnd entladenen Kondensatoren (1 und 2) entgegengesetzte Polaritäten darstellen.

17. Vorrichtung nach Anspruch 14 oder 15, **dadurch gekennzeichnet, dass** die Anschlüsse (1' und 2') der abwechselnd entladenen Kondensatoren (1 und 2) gleiche Polaritäten darstellen, wobei die sich ergebenden Pulse dafür ausgebildet sind, über eine Brückenschaltung (7) an den Patienten (5) angelegt zu werden, die die Impulse mit entgegengesetzten Phasen abgeben kann.

18. Vorrichtung nach einem beliebigen der Ansprüche 14 bis 17, **dadurch gekennzeichnet, dass** sie Mittel zum Verändern des Formfaktors des Signals zur Aufteilung oder Zerschneidung der Welle umfasst.

19. Vorrichtung nach dem vorhergehenden Anspruch, **dadurch gekennzeichnet, dass** sie Mittel zum Verändern des Formfaktors des Signals zur Aufteilung oder Zerschneidung der Welle umfasst, um die an den Patienten angelegte Defibrillierungsenergie zu verändern.

20. Vorrichtung nach dem vorhergehenden Anspruch, **dadurch gekennzeichnet, dass** sie einerseits aus mindestens einem Kondensator zur Speicherung der elektrischen Defibrillierungsenergie besteht und andererseits aus mindestens 2, durch eine Steuerschaltung gesteuerten Unterbrechern oder Umschaltern besteht, um eine Reihe von Pulsen in der Form einer mindestens zweiphasigen Welle zu erzeugen, deren aufeinanderfolgende Phasen mit den entgegengesetzten Polaritäten mit einer höheren Frequenz aufgeteilt oder zerschnitten werden als die Frequenz der aufeinanderfolgenden Phasen, nach einem Formfaktor, der variiert.
